# EUROPÄISCHE PATENTSCHRIFT

(11) **EP 1 161 936 B1**
(45) Veröffentlichungstag und Bekanntmachung des Hinweises auf die Patenterteilung: **15.10.2003**
(21) Anmeldenummer: 01113895.5
(22) Anmeldetag: 08.06.2001
(51) Int. Cl.: A61K 7/135, A61K 7/06

(54) **Verfahren zum Blondieren von menschlichen Haaren Phthalimidoperoxyhexansäure enthaltend**
Process for bleaching human hair comprising phthalimido-peroxy-hexanoic acid
Procédé pour la décoloration des cheveux humains comprenant l'acide phthalimidopéroxyhexanoique

(30) Priorität: 10.06.2000 DE 10028965
(43) Veröffentlichungstag der Anmeldung: 12.12.2001
(73) Patentinhaber: KPSS-Kao Professional Salon Services GmbH, 64297 Darmstadt (DE)
(72) Erfinder: Eberling, Walter, 64560 Riedstadt-Crumstadt (DE)

(56) Entgegenhaltungen:
- EP-A- 0 895 777
- WO-A-96/05802
- WO-A-98/27943
- US-A- 5 550 256

## Beschreibung

Die vorliegende Erfindung betrifft ein neues Verfahren zum intensiven, jedoch gleichwohl schonenden Blondieren und Aufhellen von menschlichen Haaren.

Das Blondieren menschlicher Haare besteht üblicherweise aus folgenden Verfahrensschritten:

Homogenes Vermischen einer wasserfreien Zubereitung, vorzugsweise eines Pulvers, das mindestens ein festes Peroxidsalz, insbesondere Ammonium-, Kaliumund/oder Natriumpersulfat oder Erdalkaliperoxide enthält, mit einer wäßrigen Wasserstoffperoxid-Lösung, Aufbringen dieser Zusammensetzung auf das Haar, und Ausspülen nach vollendeter Blondierung. Dabei ist seit längerem bekannt, daß die Verwendung der im Hinblick auf die Blondierung wirksamsten Bestandteile, insbesondere der Persulfate, vor allem des Ammoniumpersulfats, in den erforderlichen höheren Konzentrationen zu Haarschädigungen und gegebenenfalls auch Hautreizungen führen kann.

Es wurde bereits versucht, dieses Problem durch (Teil-)Substitution der Persulfate zu lösen, was bisher jedoch nicht in zufriedenstellender Weise gelungen ist.

Es wurde nunmehr gefunden, und das ist Gegenstand der vorliegenden Erfindung, daß durch die Verwendung von Phthalimidoperoxyhexansäure eine befriedigende Blondierwirkung erreicht wird, ohne eine Haarschädigung oder Hautirritation hervorzurufen.

Gegenstand der Erfindung ist somit ein Verfahren zum Blondieren von menschlichen Haaren durch Behandeln derselben mit einer Zubereitung, die durch Vermischen einer wasserfreien Zusammensetzung (A), die mindestens 10 Gew.-%, vorzugsweise etwa 20 bis 75, insbesondere etwa 30 bis 60, Gew.-%, Phthalimidoperoxyhexansäure enthält, mit einer wäßrigen Wasserstoffperoxidzusammensetzung (B) erhalten wurde.

Die Verwendung von Phthalimidoperoxyhexansäure als technisches Bleichmittel ist an sich bekannt.

Es war jedoch überraschend und nicht erwartbar, daß diese Verbindung in Kombination mit wäßrigen Wasserstoffperoxid-Zubereitungen eine haarschonende Blondierwirkung auf menschlichem Haar bewirken würde.

Bei wasserfreien Zusammensetzungen (A) handelt es sich vorzugsweise um Pulver, obwohl grundsätzlich auch Suspensionen bzw. Dispersionen der in der DE-A 38 14 356 beschriebenen Art einsetzbar sind.

Gemäß einer bevorzugten Ausführungsform der Erfindung werden die Blondier- und Aufhellungspulver als staubfreie Produkte verwendet, die als Granulate bzw. Agglomerate vorliegen.

Es kann sich dabei beispielsweise um die in der EP-A 560 088 beschriebenen Produkte, die ein Öl bzw. ein flüssiges Wachs enthalten, handeln, wobei das Aufbringen dieses Öls oder Wachses zweckmäßigerweise durch Aufsprühen erfolgt.

Auch staubfreie Blondierpulver, die entsprechend der EP-A 630 643 durch Agglomerieren der pulverförmigen Ausgangsbestandteile mittels Aufsprühen von geschmolzenen Wachsen oder C₁₀-C₁₈-Fettsäurealkanolamiden oder Verschmelzen mit denselben bei erhöhter Temperatur hergestellt wurden, sind erfindungsgemäß geeignet.

Neben dem aktiven Bestandteil enthalten die Blondiermittelzusammensetzungen noch die in solchen Mitteln üblichen Bestandteile:

Wenn es sich um ein Pulver handelt, insbesondere inerte pulverförmige Trägerstoffe, beispielsweise pyrogenes Siliciumioxid, Stärkepulver, etc., Alkalisierungsmittel wie Natriummetasilikat, oberflächenaktive Substanzen, Bindemittel, etc.; zur Vermeidung von Wiederholungen wird auf die einschlägigen Standardwerke, beispielsweise K. Schrader, "Grundlagen und Rezepturen der Kosmetika, 2. Auflage (1989, Hüthig Buchverlag), S.815 bis 823, verwiesen.

Die Mitverwendung weiterer Peroxide in untergeordneten Mengen, z.B. Natrium- und Kaliumpersulfat, Magnesiumperoxid, Melaminperoxid oder Harnstoffperoxid, ist im Prinzip möglich; der Anteil an Ammoniumpersulfat sollte jedoch 0 bis maximal 1 Gew.-% betragen.

Gemäß einer bevorzugten Ausführungsform der Erfindung können die Blondiermittel noch 0,1 bis 10 Gew.-%, berechnet auf die Gesamtzusammensetzung, eines oder mehrerer Ammoniumsalze enthalten.

Geeignete Ammoniumsalze sind dabei Ammoniumcarbonat, Ammoniumhydrogencarbonat, Ammoniumcarbamat, Ammoniumchlorid, Ammoniumsutfat, Ammoniumphosphate, Ammoniumnitrat, Ammoniumbromid, Ammoniumjodid, Ammoniumthiosulfat, Ammoniummolybdat, Ammoniumvanadat, Ammoniumsulfamat, Ammoniumcitrat, Ammoniumsalicylat, Ammoniumvalerat, Ammoniumartrat, Ammoniumbenzoat, Ammoniumacetat, Ammoniumformiat und Ammoniumlactat.

Bevorzugt sind hierbei die Ammoniumphosphate wie NH₄H₂PO₄, (NH₄)₂HPO₄, (NH₄)₂NaPO₄, NaNH₄HPO₄ oder NH₄Na₂PO₄, Ammoniumchlorid, Ammoniumsulfat und Diammoniumhydrogencitrat sowie Ammoniumchlorid, vorzugsweise in einer Menge von 0,1 bis 5 Gew.-%.

Der Anteil der Perverbindungen insgesamt liegt vorzugsweise zwischen etwa 5 und etwa 75, insbesondere etwa 25 und etwa 60 Gew.-%, bezogen auf das Aufhellungsund Blondierpulver.

Die Teilchengrößen der erfindungsgemäßen Blondiermittel liegen in der Regel unterhalb 1 mm, vorzugsweise unterhalb 500 Mikron, beispielsweise bei weniger als 400 Mikron, insbesondere etwa 25 bis etwa 100 µm, was eine ausgezeichnete Verarbeitbarkeit, d.h. Mischbarkeit mit einer wäßrigen Wasserstoffperoxid-Lösung vor Applikation auf das menschliche Haar gewährleistet.

Die Anwendung des Mittels geschieht in an sich bekannter und üblicher Weise:

Das pulverförmige Aufhellungsmittel wird mit einer 6- bis 12-prozentigen Wasserstoffperoxid-Lösung, vorzugsweise in einem Verhältnis von etwa 1 Gewichtsteil des Pulvers zu etwa 0,5 bis etwa 4, insbesondere etwa 1 bis etwa 2,5 Gewichtsteilen der Peroxid-Lösung oder -Lotion homogen vermischt und anschließend etwa 10 bis etwa 60, insbesondere etwa 20 bis 30 Minuten, je nach Temperatur, auf dem Haar zur Einwirkung gebracht.

Die Anwendung kann auch in der Weise geschehen, daß das Blondierpulver und die Wassertoffperoxidlösung mit einer Cremegrundlage vermischt werden, und diese homogene Mischung dann auf das Haar aufgetragen wird.

Vorzugsweise ist die Zusammensetzung so eingestellt, daß beim Mischen mit wäßriger Wasserstoffperoxid-Lösung -Lösung ein pH-Wert der gebrauchsfertigen Mischung von etwa 8 bis etwa 11,5, insbesondere zwischen 9 bis 11, erhalten wird.

Die folgenden Beispiele dienen der Illustration der Erfindung.

### Beispiel 1

| **a) Aufhellungspulver** | |
|---|---|
| Siliciumdioxid | 15,8 (Gew.-%) |
| Ammoniumchlorid | 14,7 |
| Natriumcarbonat | 10,0 |
| Natriumetasilicat | 2,3 |
| Phthalimidoperoxyhexansäure | 42,0 |
| Natriumpersulfat | 15,2 |

Diese Zusammensetzung wird im Gewichtsverhältnis 1:2,5:2,5 mit einer H₂O₂-Lotion b) und einer Cremegrundlage c) vermischt:

| **b) Wasserstoffperoxid-Lotion** | |
|---|---|
| Wasserstoffperoxid | 6,0 (Gew,-%) |
| Cetylstearylalkohol | 1,7 |
| Phosphorsäure | 0,5 |
| Natriumlaurylsulfat | 0,2 |
| Dinatriumhydrogenphosphat | 0,1 |
| Salicylsäure | 0,1 |
| Wasser ad | 100,0 |

| **c) Cremegrundlage** | |
|---|---|
| Cetylstearylalkohol | 11,0 (Gew.-%) |
| Oleth-5 | 5,0 |
| Ölsäure | 2,5 |
| Stearamide MEA | 2,3 |
| Cocoamide MEA | 2,3 |
| Natriumcetylstearylsulfat | 1,2 |
| 1,2-Propylenglykol | 1,0 |
| 1,2-Propylenglykolstearat | 0,6 |
| Natriumlaurylsulfat | 0,5 |
| Weizenproteinhydrolysat | 0,9 |
| Panthenol | 0,6 |
| Parfum | 0,4 |
| Komplexbildner | 0,2 |
| Wasser ad | 100,0 |

Der pH-Wert dieser Mischung lag bei 9,3.

Bei der Applikation dieser Mischung wurde eine ausgezeichnete Blondierwirkung festgestellt; eine feststellbare Haarschädigung trat an Haarsträhnen aus ungeschädigtem Menschenhaar selbst nach viermaliger aufeinanderfolgender Anwendung nicht auf.

### Beispiel 2

| Blondierpulver | |
|---|---|
| Kieselgur | 36 (Gew.-Teile) |
| Natriumcarboxymethylcelluslose | 25 |
| Hydroxyethylcellulose | 16 |
| Natriumlaurylsulfat | 25 |
| Natriumstearat | 16 |
| Eiweißhydrolysat | 6 |
| Stärke | 10 |
| Natriumcarbonat | 8 |
| Natriummetasilikat | 90 |
| Polyvinylpyrrolidon K90 | 32 |
| Kokosfettsäuremonoethanolamid | 98 |
| (NH₄)₂HPO₄ | 20 |
| Magnesiumperoxid | 38 |
| Kaliumpersulfat | 80 |
| Phthalimidoperoxyhexansäure | 500 |
| pH-Wert nach dem Anrühren mit 6%iger H₂O₂-Lösung im Verhältnis 1:1 | 9,5 |

Auch bei Anwendung dieser Mischung wurde eine ausgezeichnete Blondierwirkung, verbunden mit einer nicht meßbaren Haarschädigung, erhalten.

### Beispiel 3

| **Staubfreies Blondierpulver** | |
|---|---|
| Siliciumdioxid (Diatomenerde) | 3,2 (Gew.-%) |
| Siliciumdioxid (pyrogenes SiO₂) | 5,3 |
| Natriumcarboxymethylcellulose | 3,5 |
| Harnstoff | 2,0 |
| Natriumlauroylsarkosinat | 0,8 |
| Natriumstearat | 1,2 |
| Natriumcarbonat | 1,0 |
| Natriummetasilikat | 6,0 |
| Stärkepulver | 3,5 |
| Phthalimidoperoxyhexansäure | 58,0 |
| Magnesiumperoxid | 4,0 |
| Paraffinöl (Paraffinum perliquidum, DAB 9) | 11,5 |

Es wird ein staubfreies Pulver erhalten, das mit einer bekannten 9%igen H₂O₂-Lösung im Gewichtsverhältnis 1:1,5 gut angerührt werden kann. 99% der Teilchen hatten einen Durchmesser von < 400 Mikron.

Die Blondierwirkung ist ausgezeichnet; eine Haarschädigung war nicht nachweisbar.

Die Herstellung des Pulvers erfolgte durch Aufsprühen des Paraffinöls auf die Pulvergrundmasse bei etwa 20°C im Wirbelsprühverfahren.

### Beispiel 4

| **Staubfreies Blondier-Agglomerat** | |
|---|---|
| Siliciumdioxid (Diatomenerde) | 3,2 (Gew.-%) |
| Siliciumdioxid (pyrogenes SiO₂) | 5,3 |
| Natriumcarboxymethylcellulose | 3,5 |
| Harnstoff | 2,0 |
| Natriumlauroyisarkosinat | 0,8 |
| Natriumstearat | 1,2 |
| Natriumcarbonat | 1,0 |
| Natriummetasilikat | 6,0 |
| Stärkepulver | 3,5 |
| Phthalimidoperoxyhexansäure | 40,0 |
| Kaliumpersuifat | 14,5 |
| Magnesiumperoxid | 4,0 |
| Cocosmonoethanolamid | 15,0 |

Es wurde ein staubfreies wasserlösliches bzw. -dispergierbares Pulver enthalten, das mit einer bekannten 9%igen H₂O₂-Lösung im Gewichtsverhältnis 1:1,5 gut angerührt werden konnte.

99% der Teilchen hatten einen Durchmesser von < 400 Mikron.

Die am Haar erzielte Blondierwirkung war ausgezeichnet; eine Haarschädigung war nicht erkennbar.

Die Herstellung des Pulvers erfolgte durch Aufheizen der obengenannten Mischung auf 70°C bis 75°C in einem Wirbelstromgenerator und anschließendes Abkühlen.

## Patentansprüche

1. Verfahren zum Blondieren von menschlichen Haaren, wobei eine wasserfreie Zusammensetzung (A), die mindestens 10 Gew.-% Phthalimidoperoxyhexansäure enthält, mit einer wäßrigen Wasserstoffperoxidzusammensetzung (B) vermischt, die Mischung auf menschliches Haar aufgebracht und nach erfolgter Einwirkung aus dem Haar ausgespült wird.

2. Verfahren nach Anspruch 1, daß die wasserfreie Zusammensetzung (A) aus einem Pulver besteht und etwa 20 bis 75 Gew.-% Phthalimidoperoxyhexansäure, berechnet auf das Pulver, enthält.

## Claims

1. Process for the bleaching of human hair, wherein a water-free composition (A), comprising at least 10 % by weight of phthalimidoperoxyhexanic acid, is mixed with an aqueous hydrogen peroxide compositio (B), the mixture is applied onto human hair and rinsed out of the hair after completed processing.

2. Process according to claim 1, wherein the aqueous composition (A) consists of a powder and contains about 20 % to 75 % by weight, calculated to the powder, of phthalimidoperoxyhexanic acid.

## Revendications

1. Procédé destiné à la décoloration des cheveux humains, par lequel une composition non aqueuse (A) comprenant au moins 10% en poids d'acide phthalimidopéroxyhexanoïque est mélangée à une solution aqueuse de peroxyde d'hydrogène (B), ledit mélange étant appliqué sur les cheveux humains pour être ensuite, après l'avoir laissé agir, rincé.

2. Procédé selon la revendication 1 **caractérisée en ce que** la composition non aqueuse (A) est une poudre et comprend environ 25 à 75% en poids d'acide phthalimidopéroxyhexanoïque par rapport à la poudre.
